# EUROPEAN PATENT SPECIFICATION

(11) **EP 2 560 574 B1**
(45) Date of publication and mention of the grant of the patent: **25.02.2015**
(21) Application number: 11721370.2
(22) Date of filing: 19.04.2011
(51) Int. Cl.: A61F 2/00

(54) **MINIMALLY INVASIVE SLING FOR THE SURGICAL TREATMENT OF FEMALE URINARY STRESS INCONTINENCE**
MINIMAL INVASIVE SCHLINGE ZUR CHIRURGISCHEN BEHANDLUNG VON WEIBLICHER BELASTUNGSHARNINKONTINENZ
BANDELETTE SOUS URÉTRALE MINIMALEMENT INVASIVE POUR LE TRAITEMENT CHIRURGICAL DE L'INCONTINENCE URINAIRE D'EFFORT CHEZ LA FEMME

(30) Priority: 19.04.2010 IT TO20100314
(43) Date of publication of application: 27.02.2013
(73) Proprietor: Herniamesh S.R.L., 10034 Chivasso (Torino) (IT)
(72) Inventor: CREPALDI, Pier, Aldo, I-13046 Livorno Ferraris (Vercelli) (IT); LAMBERTI, Roberta, I-10056 Ulzio (Torino) (IT); PIROLI TORELLI, Donato, I-81100 Napoli (IT); TRABUCCO, Ermanno, Muttontown, New York 11791 (US)
(74) Representative: Gerbino, Angelo
(86) International application number: PCT/IB2011/051686
(87) International publication number: WO 2011/132141

(56) References cited:
- WO-A2-2006/102477
- WO-A2-2007/106520
- US-A1- 2002 028 980
- US-A1- 2005 267 325
- US-A1- 2007 021 649

## Description

The present invention relates to a device for treating urinary incontinence, a common disorder which predominantly affects women. It has been estimated that more than 13 million individuals suffer from urinary incontinence in the United States, 85% of them women. Regarding the prevalence of urinary incontinence in women from four different European countries (France, Germany, Spain and the UK), 35% of women interviewed who wished to provide an answer reported involuntary losses of urine in the 30 days preceding the interview. The type of incontinence was predominantly stress incontinence (S. Hunskaar et al - The prevalence of urinary incontinence in women in four European countries - BJU International, Volume 93 Issue 3, Pages 324-330, published online: 4 Feb 2004). Type I and II stress incontinence is caused by urethral hypermobility, a condition in which the pelvic floor is weakened or damaged, leading to the lowering of the neck of the bladder and/or the proximal urethra in response to an increase in intra-abdominal pressure. This pressure may be due to various routine daily activities such as laughing, sneezing, coughing, lifting weights, walking or getting up from a seat. The result is an inadequate sphincter response of the urethra and a consequent loss of urine. Biological factors which may cause hypermobility include insufficient endopelvic muscle tone (due to age or limited physical activity), stretching caused by injury to the endopelvic fascia, due to pregnancy for example, separation of muscle and ligament (fascia and arcus tendineus), or hormone (oestrogen) deficiency.

In order to increase the urethral closure pressure and thus mitigate involuntary loss of urine, a support is provided under the urethra by a surgical procedure.

The surgical treatment most commonly used at present for female urinary stress incontinence requires the implantation of what is known as a "sling" in a sub-urethral position. Conventionally, slings are inserted under the urethra in order to provide a bearing and support surface to limit the prolapse of the endopelvic muscles as far as possible at the time when the urethral sphincter is subject to compression. The complexity of the surgical procedure and the technical difficulties regarding the anatomical positioning of the sling have caused problems for surgeons and patients for some time. Some procedures for implanting urethral supports require multiple incisions in the patient, and, in some cases, the sling is also tensioned from outside the patient's body after the operation.

For many other commonly implanted slings, however, fixing or anchoring to bone, tissues, skin or muscles by screwing or suturing is required. In fact, there is no need to provide any particular means of tensioning or fixing the sling to achieve continence, because the urethra is not stabilized and supported by the sling itself, but rather by the reaction of the tissue fibres which develop through the pores of the sling. The process of fibrosis promotes the adhesion of the sling to the adjacent tissues, and this is the basis of the suspension mechanism used in the tension-free procedure (D. Piroli Torelli et al. - SUS for the correction of stress urinary incontinence: towards more and more simplified surgery - Urogynaecologia International Journal 2008; 22; 8: 5-15).

Indeed, the tension-free TVT (trans-vaginal tape) or TOT (trans-obturator tape) surgical procedures are most commonly used, although even in these cases multiple incisions are required and insertion needles have to be used for the insertion of the device. The sling can normally be up to 45 cm long: consequently the excess material not directly concerned with urethral support extends through the abdominal wall, in the case of the TVT method, or the obturator foramen, in the case of the TOT method, and remains permanently implanted as a possible source of infection and discomfort throughout the patient's lifetime.

Technical problems and serious complications can arise during the passage of the needles. For example, some typical complications of the TVT method include possible perforation of the bladder, requiring cystoscopy, perforations of the intestine, nerve and blood vessel damage which may cause intra-operative bleeding, and death (Daniel Rapoport, MD, Howard N. Fenster, MD, Jamie E. Wright, MD - Reported complications of tension-free vaginal tape procedures: A review - BCMJ, Vol. 49, No. 9, November 2007, page(s) 465-524).

The TOT method too can give rise to potential complications: these include occasional intra-operative haemorrhages, pain and discomfort in the patient, infections, bleeding and post-operative urethral obstruction (Neuman M. - TVT-Obturator: Short-term data on an operative procedure for the cure of female stress urinary incontinence performed on 300 patients - Eur Urol 2007; 51: 1083-1087; Rezapour M, Novara G, Meier PA et al. - A three-month pre-clinical trial to assess the performance of a new TVT-like mesh (TVTx) in a sheep model - Int Urogynecol J Pelvic Floor Dysfunc 2007; 18: 183-187). The pain which develops in the inguinal and hip area is a consequence of the lithotomy position with the legs wide apart which the patient has to assume during the operation in order to provide better access to the obturator foramen; this complication becomes more serious in the case of women with coxarthrosis or reduced joint function.

The prior document WO2008/067 317 describes a sling comprising an oblong median portion and end portions for anchoring to the tissues, in which the sling is implanted surgically. This median portion is made from biocompatible polymer mesh and is associated with bioabsorbable polymer end portions having saw-tooth or pointed protrusions. The end portions are rather bulky and consequently invasive in relation to the tissues in which they are to be implanted, and are therefore made from bioabsorbable polymer in such a way that they can disappear over time.

Another prior document, WO2005/122 954, describes a sling formed by a mesh folded back on itself at the ends to form corresponding pockets for the engagement of instruments for positioning the device which do not perforate the abdominal wall.

US-2005/0267325 describes generic implantable surgical articles, and is mainly concerned with methods of joining different materials or layers of the same material. Drawings in US-2005/0267325 illustrate a sling comprising a first mesh having end parts whose two faces are covered by a corresponding second mesh which is Y-shaped, the branches of the Y being adjacent to the corresponding faces of the associated end part of the first mesh, and the stem of the Y forming the corresponding end portion of the sling. Thus the first mesh does not extend from one end portion of the sling to the other. The two parts of the Y-shaped second mesh are joined to the first mesh by a corresponding polymer rivet passing through an aperture perpendicular to the general plane of the sling, and in order to achieve this it is typically necessary to carry out an ad hoc process which may promote the formation of gaps in the meshes. It should be noted that this structure with three layers joined by a rivet, which is the essence of what is described in US-2005/0267325, makes the ends of the sling rigid and bulky.

US-2002/0028980 describes an implantable article comprising a base portion, from one end of which two engagement portions extend independently of each other. This article has a Y-shaped profile, in other words a profile which is not compact. Additionally, US-2002/0028980 simply asserts that the article described therein can comprise, in a non-limiting way, pores having dimensions ranging from 1.016 to 1.397 mm, without mentioning the average dimension or the possibility of additionally having pores of different dimensions, but solely stating that the choice of pore dimensions is dictated by considerations relating to the fixing to the surrounding tissues. US2007/0021649 A1 discloses a sling having an oblong median portion, a first mesh, a second member joined to and superimposed on first mesh near the end portions of said first mesh, wherein the first mesh extends through said median portion.

The object of the present invention is therefore to provide a device which is improved with respect to the prior art, and which, in particular, is free of the aforementioned disadvantages and can be used for simpler and safer surgical procedures.

According to the invention, this object is achieved by means of a sling as disclosed in the appended claims comprising an oblong median portion and anchoring end portions, with a first mesh of synthetic, biocompatible, non-absorbable polymer extending from one end portion to the other through the median portion, and a second mesh of synthetic, biocompatible, non-absorbable polymer having average porosity lower than the average porosity of the first mesh, but nevertheless greater than 100 µm, being joined to and superimposed on the parts of the first mesh belonging to the end portions.

In the present description, the term "average porosity of a mesh" denotes, in particular, the arithmetic mean of the dimensions of the pores present between the filaments, which is determined by the conditions and by the type of weaving.

The sling according to the invention therefore combines a first mesh (such as that described in Italian patent application MI2009A001186 ) having high porosity, reduced elasticity and a high axial breaking load, with a second mesh which stiffens the end portions which still remain flexible and macroporous to some extent. Macroporosity is a key attribute of the sling according to the invention, because it not only promotes fibroblast infiltration which is the principle on which strong and permanent anchorage is based, but also minimizes the onset of any infection. This is because bacteria can invade all surgical meshes because their average diameter is about 1 µm, but if the pores of the mesh are large enough to allow the infiltration of macrophages, which are immune cells with dimensions of about 10 µm, responsible for the phagocytosis of bacteria, then defence against infection can take place. Since the average porosity of the end portions of the sling according to the invention is greater than 100 µm, as mentioned above, the sling has the advantage of permitting both macrophage and fibroblast infiltration.

Anchoring in the immediate post-operative period takes place by means of the stiffened end portions which provide a strong attachment in the periurethral connective tissue. Another factor helping to keep the sling in position is that it is subject to two opposing pressures, one from the outside to the inside, created by the vaginal mucosa and the underlying fascia, and the other from the inside to the outside, created by the intra-abdominal pressure, both acting within an anatomically closed space. The positioning of the sling, which is not attached in a fixed position to the obturator membrane, can thus be described actually as "tension free". A few hours after the operation, the pores of the sling are infiltrated by fibrous tissue neoformations which also assist with the anchorage.

Because of its specific structure, the overall length of the sling according to the invention can be shorter than that of similar known devices, whose length is such that the membrane is perforated. Conversely, the length of the sling according to the invention is about 66 mm, thus permitting pre-obturator positioning which follows the same direction as the aforementioned TOT slings of the prior art.

Considered as a whole, the sling according to the invention represents a markedly smaller quantity of foreign matter for the body in which it is inserted, but it can still be implanted in an adequate and permanent way.

Furthermore, the sling according to the invention enables the advantageous "single incision" operating procedure to be used, thus reducing the passage of surgical instruments to a minimum while also limiting intra-operative risks and post-operative pain.

Other advantages and characteristics of the present invention will become clear from the following detailed description which is given by way of non-limiting example with reference to the attached drawing, in which:
Figure 1 is a plan view of the sling according to the invention, and
Figure 2 is a view in side elevation of the sling of Figure 1.

A sling for supporting the urethra comprises an oblong median portion 10 and anchoring end portions 12. For the purpose of definition, and as clearly shown in the drawings, the end portions 12 necessarily include the areas of the sling farthest from the centre.

The median portion has a shape which is generally tapering from the centre towards the ends, and in particular it is formed by a substantially rectangular central part 14 with a perimeter formed by a pair of short sides and a pair of long sides, and by end parts 16 of substantially trapezoidal shape, the longer bases of which correspond to the short sides of the central part.

Each end portion 12 is substantially arrow-shaped with a tip facing outwards, the width at the base of each arrow being greater than the maximum width of the median portion 10.

A first mesh 18 extends from one end portion 12 to the other through the whole median portion 10. A corresponding second mesh 20 having an average porosity smaller than that of the first mesh 18 is superimposed on the whole extension of the parts of the first mesh 18 belonging to the end portions 12. The two meshes 18 and 20 are joined to each other continuously over the whole contact surface, for example by welding or stitching or by any other method which does not obstruct their pores, preferably by ultrasonic welding.

Thus the meshes 18 and 20 are joined without the need for any additional bulky elements such as rivets or the like, which would stiffen the sling and make it unwieldy, and in this way the sling is provided with a compact configuration.

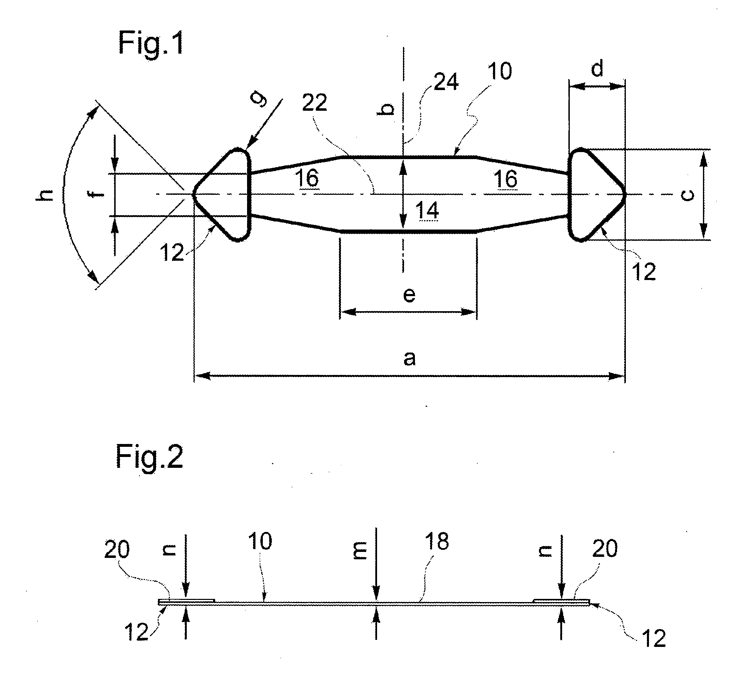

It should also be noted that, as Figure 2 clearly shows, the second mesh 20 covers only one face of each of the end portions of the first mesh 18 which coincide with the end portions 12 of the sling as a whole, while the opposite faces of the end portions of the first mesh 18 are left free.

Considered as a whole, the sling is symmetrical about a longitudinal axis 22 and also about a transverse axis 24 lying in its general plane, and is constituted by the meshes 18 and 20.

Advantageously, the first mesh 18 has a nominal density in the range from 30 to 60 g/m², preferably 48 g/m², and an average porosity in the range from 800 µm to 1200 µm, preferably 1000 µm, while the second mesh 20 has a nominal density in the range from 100 to 160 g/m², preferably 127 g/m², and an average porosity in the range from 500 µm to 900 µm, preferably 700 µm.

The first and second meshes 18 and 20 can be formed independently from filaments of any non-absorbable biocompatible synthetic polymer, for example a polymer chosen from the group composed of homopolymers and copolymers of polypropylene, polyethylene, polyester, polyamide, partially or totally fluorinated polymers and mixtures thereof. The filaments of the meshes 18 and 20 can also be covered with any biocompatible material (whether bioreabsorbable or not), provided that the aforementioned porosity parameters are adhered to. In particular, the mesh 18 can be made from 80 µm PP monofilament, and the mesh 20 can be made from 180 µm PP monofilament.

By way of example, the sling may have an overall length "a" in the range from 55 to 75 mm, preferably 66 mm, while each end portion 12 can have a length "d" in the range from 5 to 15 mm, preferably 8.5 mm.

Also by way of example, the shorter base of each end part 16 of the median portion 10 can have a length "f" in the range from 5 to 9 mm, preferably 6.5 mm, while each long side can have a length "e" in the range from 10 to 40 mm, preferably 21 mm, and each short side can have a length "b" in the range from 9 to 12 mm, preferably 11 mm.

Advantageously, each arrow-shaped end portion 12 has a width at the base "c" in the range from 11 to 16 mm, preferably 13.5 mm, and a vertex angle "h" in the range from 75° to 105°, preferably 90°. Considered as a whole, each arrow is shaped in the form of an isosceles triangle with rounded angles "g".

In preferred embodiments, the thickness "m" of the median portion 10 is in the range from 0.25 to 0.35 mm, preferably 0.3 mm, while the thickness "n" of the end portions 12 due to the superimposition of the meshes 18 and 20 is in the range from 0.4 to 0.6 mm, preferably 0.5 mm.

For the surgical implantation of a sling of the type described above, a suburethral vaginal incision is initially made with a length of about 1 cm, after which two tunnels with a length of about 3.5 cm are made to the right and left of the urethra, following the path used in the TOT method, until contact is made with the obturator membranes without perforating them. One of the arrow-shaped end portions 12 is then folded back on itself and is grasped with a curved Klemmer Mosquito forceps so that it can be inserted into the first transobturator tunnel. The same operations are then carried out on the other end portion 12 to insert it into the second tunnel.

Finally, a colporrhaphy is performed on the vaginal incision, with one or two separate introflecting stitches.

Naturally, the principle of the invention remaining the same, the details of construction and the forms of embodiment may be varied widely with respect to those described, which have been given purely by way of example, without thereby departing from the scope of the invention as defined in the attached claims.

## Claims

1. Sling for supporting the urethra, comprising an oblong median portion (10) and anchoring end portions (12), **characterized in that** a first mesh (18) of synthetic, biocompatible, non-absorbable polymer extends from one end portion (12) to the other through the median portion (10), a second mesh (20) of synthetic, biocompatible, non-absorbable polymer having average porosity lower than the average porosity of the first mesh (18), but nevertheless greater than 100 µm, being joined to and superimposed on the parts of the first mesh (18) belonging to the end portions (12).

2. Sling according to Claim 1, wherein said first mesh (18) has a nominal density in the range from 30 to 60 g/m², preferably 48 g/m², and an average porosity in the range from 800 to 1200 µm, preferably 1000 µm.

3. Sling according to Claim 1 or 2, wherein said second mesh (20) has a nominal density in the range from 100 to 160 g/m², preferably 127 g/m², and an average porosity in the range from 500 to 900 µm, preferably 700 µm.

4. Sling according to any one of the preceding claims, wherein the thickness of the median portion (10) is in the range from 0.25 to 0.35 mm, preferably 0.3 mm, whereas the thickness of the end portions (12) is in the range from 0.4 to 0.6 mm, preferably 0.5 mm.

5. Sling according to any one of the preceding claims, wherein said second mesh (20) is joined to the first mesh (18) over the whole extension of the end portions (12) preferably by welding or sewing, particularly by ultrasonic welding.

6. Sling according to any one of the preceding claims, having an overall length in the range from 55 to 75 mm, preferably 66 mm, each end portion (12) having a length in the range from 5 to 15 mm, preferably 8.5 mm.

7. Sling according to any one of the previous claims, wherein said median portion (10) has a generally tapering shape from the centre towards the ends.

8. Sling according to Claim 7, wherein said median portion (10) is formed by a substantially rectangular central part (14) whose perimeter is formed by a pair of shorter sides and a pair of longer sides, and by end parts (16) having a substantially trapezoidal shape, whose longer bases correspond to the shorter sides of the centre part (14).

9. Sling according to Claim 8, wherein the shorter base of each end part (16) of the median portion (10) has a length in the range from 5 to 9 mm, preferably 6.5 mm.

10. Sling according to Claim 8 or 9, wherein each longer side has a length in the range from 10 to 40 mm, preferably 21 mm, and each shorter side has a length in the range from 9 to 12 mm, preferably 11 mm.

11. Sling according to any one of the preceding claims, wherein each end portion (12) is substantially arrow-shaped with a tip facing outwards, the width at the base of each arrow being greater than the maximum width of the median portion (10).

12. Sling according to Claim 11, wherein each arrow has a width at the base in the range from 11 to 16 mm, preferably 13.5 mm, and a vertex angle in the range from 75° to 105°, preferably 90°.

13. Sling according to any one of the preceding claims, wherein said first and second meshes (18, 20) are independently formed from filaments of polymer chosen from the group composed of homopolymers and copolymers of polypropylene, polyethylene, polyester, polyamide, partially or totally fluorinated polymers and mixtures thereof.

14. Sling according to Claim 13, wherein said filaments have a coating of biocompatible material.

15. Sling according to any one of the preceding claims, wherein said second mesh (20) covers only one face of each of the end portions of said first mesh (18), while the opposite face is left free.

## Patentansprüche

1. Schlinge zur Stützung der Harnröhre, die einen länglichen mittleren Abschnitt (10) und verankernde Endabschnitte (12) umfasst, **dadurch gekennzeichnet, dass** sich ein erstes Gewebe (18) aus einem synthetischen, biologisch verträglichen, nicht-absorbierbaren Polymer von einem Endabschnitt (12) über den mittleren Abschnitt (10) zu dem anderen Endabschnitt erstreckt, wobei ein zweites Gewebe (20) aus einem synthetischen, biologisch verträglichen, nicht-absorbierbaren Polymer, das eine durchschnittliche Porosität aufweist, die niedriger ist als die durchschnittliche Porosität des ersten Gewebes (18), jedoch größer als 100 µm ist, mit den Teilen des ersten Gewebes (18), die zu den Endabschnitten (12) gehören, verbunden ist und diesen überlagert ist.

2. Schlinge nach Anspruch 1, bei der das erste Gewebe (18) eine Nenndichte im Bereich von 30 bis 60 g/m², vorzugsweise 48 g/m², und eine durchschnittliche Porosität im Bereich von 800 bis 1200 µm, vorzugsweise 1000 µm, aufweist.

3. Schlinge nach Anspruch 1 oder 2, bei der das zweite Gewebe (20) eine Nenndichte im Bereich von 100 bis 160 g/m², vorzugsweise 127 g/m², und eine durchschnittliche Porosität im Bereich von 500 bis 900 µm, vorzugsweise 700 µm, aufweist.

4. Schlinge nach einem der vorhergehenden Ansprüche, bei der die Dicke des mittleren Abschnitts (10) im Bereich von 0,25 bis 0,35 mm, vorzugsweise 0,3 mm, liegt, wohingegen die Dicke der Endabschnitte (12) im Bereich von 0,4 bis 0,6 mm, vorzugsweise 0,5 mm, liegt.

5. Schlinge nach einem der vorhergehenden Ansprüche, bei der das zweite Gewebe (20) mit dem ersten Gewebe (18) über die gesamte Ausdehnung der Endabschnitte (12) vorzugsweise mittels Schweißen oder Nähen, insbesondere mittels Ultraschallschweißen, verbunden worden ist.

6. Schlinge nach einem der vorhergehenden Ansprüche, die eine Gesamtlänge im Bereich von 55 bis 75 mm, vorzugsweise 66 mm, aufweist, wobei jeder Endabschnitt (12) eine Länge im Bereich von 5 bis 15 mm, vorzugsweise 8,5 mm, aufweist.

7. Schlinge nach einem der vorhergehenden Ansprüche, bei welcher der mittlere Abschnitt (10) von der Mitte zu den Enden hin im Allgemeinen eine sich verjüngende Form aufweist.

8. Schlinge nach Anspruch 7, bei welcher der mittlere Abschnitt (10) aus einem im Wesentlichen rechteckigen zentralen Teil (14), dessen Umfang durch ein Paar von kürzeren Seiten und ein Paar von längeren Seiten ausgebildet ist, und durch Endteile (16) ausgebildet ist, die im Wesentlichen eine Trapezform aufweisen, deren längere Basisabschnitte den kürzeren Seiten des zentralen Teils (14) entsprechen.

9. Schlinge nach Anspruch 8, bei welcher der kürzere Basisabschnitt jedes Endteils (16) des mittleren Abschnitts (10) eine Länge im Bereich von 5 bis 9 mm, vorzugsweise 6,5 mm, aufweist.

10. Schlinge nach Anspruch 8 oder 9, bei der jede längere Seite eine Länge im Bereich von 10 bis 40 mm, vorzugsweise 21 mm, aufweist, und jede kürzere Seite eine Länge im Bereich von 9 bis 12 mm, vorzugsweise 11 mm, aufweist.

11. Schlinge nach einem der vorhergehenden Ansprüche, bei der jeder Endabschnitt (12) im Wesentlichen pfeilförmig ist, wobei eine Spitze auswärts gerichtet ist, wobei die Breite an der Basis jedes Pfeils größer ist als die maximale Breite des mittleren Abschnitts (10).

12. Schlinge nach Anspruch 11, bei der jeder Pfeil eine Breite an der Basis im Bereich von 11 bis 16 mm, vorzugsweise 13,5 mm, und einen Scheitelwinkel im Bereich von 75° bis 105°, vorzugsweise 90°, aufweist.

13. Schlinge nach einem der vorhergehenden Ansprüche, bei der das erste und das zweite Gewebe (18, 20) unabhängig aus Fäden eines Polymers ausgebildet sind, das aus der Gruppe, bestehend aus Homopolymeren und Copolymeren von Polypropylen, Polyethylen, Polyester, Polyamid, teilweise oder vollständig fluorierten Polymeren und Gemischen davon, ausgewählt ist.

14. Schlinge nach Anspruch 13, bei der die Filamente eine Beschichtung aus einem biologisch verträglichen Material aufweisen.

15. Schlinge nach einem der vorhergehenden Ansprüche, bei der das zweite Gewebe (20) nur eine Fläche von jedem der Endabschnitte des ersten Gewebes (18) bedeckt, während die gegenüber liegende Fläche frei bleibt.

## Revendications

1. Bandelette destinée à supporter l'urètre, comprenant une partie médiane oblongue (10) et des parties d'extrémités servant à la fixation (12), **caractérisée en ce qu'**un premier filet (18) en polymère synthétique, biocompatible et non absorbable s'étend d'une partie d'extrémité (12) à l'autre à travers la partie médiane (10), un deuxième filet (20) en polymère synthétique, biocompatible et non absorbable ayant une porosité moyenne inférieure à la porosité moyenne du premier filet (18), mais néanmoins supérieure à 100 µm, qui est relié à et superposé sur les parties du premier filet (18) appartenant aux parties d'extrémités (12).

2. Bandelette selon la revendication 1, dans laquelle ledit premier filet (18) a une densité nominale comprise dans l'intervalle de 30 à 60 g/m², de préférence 48 g/m², et une porosité moyenne comprise dans l'intervalle de 800 à 1200 µm, de préférence 1000 µm.

3. Bandelette selon la revendication 1 ou 2, dans laquelle ledit deuxième filet (20) a une densité nominale comprise dans l'intervalle de 100 à 160 g/m², de préférence 127 g/m², et une porosité moyenne comprise dans l'intervalle de 500 à 900 µm, de préférence 700 µm.

4. Bandelette selon l'une quelconque des revendications précédentes, dans laquelle l'épaisseur de la partie médiane (10) est comprise dans l'intervalle de 0,25 à 0,35 mm, de préférence 0,3 mm, tandis que l'épaisseur des parties d'extrémités (12) est comprise dans l'intervalle de 0,4 à 0,6 mm, de préférence 0,5 mm.

5. Bandelette selon l'une quelconque des revendications précédentes, dans laquelle ledit deuxième filet (20) est lié au premier filet (18) sur toute l'étendue des parties d'extrémités (12) de préférence par soudage ou couture, en particulier par soudage par ultrasons.

6. Bandelette selon l'une quelconque des revendications précédentes, ayant une longueur totale comprise dans l'intervalle de 55 à 75 mm, de préférence 66 mm, chaque partie d'extrémité (12) ayant une longueur comprise dans l'intervalle de 5 à 15 mm, de préférence 8,5 mm.

7. Bandelette selon l'une quelconque des revendications précédentes, dans laquelle ladite partie médiane (10) a une forme généralement rétrécie du centre vers les extrémités.

8. Bandelette selon la revendication 7, dans laquelle ladite partie médiane (10) est formée par une pièce centrale substantiellement rectangulaire (14) dont le périmètre est formé par une paire de petits côtés et une paire de grands côtés, et par des pièces d'extrémités (16) ayant une forme substantiellement trapézoïdale, dont les grandes bases correspondent aux petits côtés de la pièce centrale (14).

9. Bandelette selon la revendication 8, dans laquelle la petite base de chaque pièce d'extrémité (16) de la partie médiane (10) a une longueur comprise dans l'intervalle de 5 à 9 mm, de préférence 6,5 mm.

10. Bandelette selon la revendication 8 ou 9, dans laquelle chaque grand côté a une longueur comprise dans l'intervalle de 10 à 40 mm, de préférence 21 mm, et chaque petit côté a une longueur comprise dans l'intervalle de 9 à 12 mm, de préférence 11 mm.

11. Bandelette selon l'une quelconque des revendications précédentes, dans laquelle chaque partie d'extrémité (12) est substantiellement en forme de flèche avec un bout tourné vers l'extérieur, la largeur à la base de chaque flèche étant supérieure à la largeur maximale de la partie médiane (10).

12. Bandelette selon la revendication 11, dans laquelle chaque flèche a une largeur à la base comprise dans l'intervalle de 11 à 16 mm, de préférence 13,5 mm, et un angle au sommet compris dans l'intervalle de 75° à 105°, de préférence 90°.

13. Bandelette selon l'une quelconque des revendications précédentes, dans laquelle lesdits premier et deuxième filets (18, 20) sont formés indépendamment à partir de filaments de polymère choisis dans le groupe comprenant les homopolymères et les copolymères de polypropylène, polyéthylène, polyester, polyamide, les polymères partiellement ou totalement fluorés et leurs mélanges.

14. Bandelette selon la revendication 13, dans laquelle lesdits filaments ont un revêtement en matériau biocompatible.

15. Bandelette selon l'une quelconque des revendications précédentes, dans laquelle ledit deuxième filet (20) ne couvre qu'une face de chacune des parties d'extrémités dudit premier filet (18), la face opposée restant libre.
